(19) Europäisches Patentamt / European Patent Office / Office européen des brevets

(11) **EP 4 279 608 A1**

(12) **EUROPEAN PATENT APPLICATION**

(43) Date of publication:
**22.11.2023 Bulletin 2023/47**

(21) Application number: **22180319.0**

(22) Date of filing: **22.06.2022**

(51) International Patent Classification (IPC):
***C12Q 1/48*** *(2006.01)*

(52) Cooperative Patent Classification (CPC):
**C12Q 1/48;** G01N 2333/91194; G01N 2800/04;
G01N 2800/20; G01N 2800/52

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **17.05.2022 US 202263364837 P
09.06.2022 US 202217806208**

(71) Applicant: **Follea International
Irvine, California 92614 (US)**

(72) Inventors:
• **Goren, Ofer A.
Cheyenne, 82001 (US)**
• **McCoy, John
Downey, 90241 (US)**

(74) Representative: **Schaafhausen Patentanwälte
PartGmbB
Prinzregentenplatz 15
81675 München (DE)**

(54) **METHOD FOR STABILIZING COLORIMETRIC ASSAY FOR USE WITH PLUCKED HUMAN HAIR**

(57)     Apparatuses are disclosed for performing colorimetric assays with plucked human hair using a device adapted for that purpose. Also disclosed are methods for stabilizing a colorimetric chemical assay so that it can be transported and used as an at-home device for conducting a colorimetric chemical assay are described.

FIG. 1

**Description**

TECHNICAL FIELD

The present disclosure relates to apparatuses for performing colorimetric assays with plucked human hair using a device adapted for that purpose. Also disclosed are methods for stabilizing a colorimetric chemical assay so that it can be transported and used as an at-home device for conducting a colorimetric chemical assay using plucked human hair.

BACKGROUND

Point-of-care (POC) diagnostic devices are commonly employed to assay biomarkers of different human health or disease states. For example, a pregnancy test is a lateral flow POC that reports the presence of the beta subunit of human chorionic gonadotropin (hCG) in urine. hCG is a biomarker produced by the trophoblastic cells of the fertilized ova. As such, the presence of hCG in urine can be reported to the user of the device by a chemical reaction that produces a visible color or change in the device indicating that the user is pregnant. Many different POC devices are currently available, commonly using bodily fluids as substrates (e.g., blood, urine or saliva). Each device has similar characteristics: they typically are portable, handheld and made of plastic. Most POC can only be used once and are considered disposable.

Currently, there exists a lab-based test for measuring the biomarker sulfotransferase 1A1 (SULT 1A1) in plucked human hair follicles (See US Patents 10,633,688 and 8,691,518). The method involves a use of a chemical composition that will spontaneously develop color over time, typically, days or weeks. This spontaneous reaction can be slowed by refrigerating the test solution. In the presence of SULT 1A1 enzymes, the reaction rate is increased (catalyzed), and a color is produced in a much shorter time (hours). The test is arranged in such a way that the amount of color produced in a given amount of time (e.g., 24 hours) is indicative of the amount of SULT 1A1 enzyme present in a hair follicle.

SULT 1A1 is an enzyme that catalyzes the transfer of a sulfo group from a donor molecule to an acceptor alcohol. The enzyme has wide human tissue distribution, including the liver and outer root sheath (ORS) of the hair follicle. Inter-individual variation in sulfonation capacity is important in determining an individual's metabolism to certain xenobiotics. Minoxidil is one drug that requires sulfonation to have efficacy. The sulfonation capacity of an individual's ORS can be used to rule out non-responders to 5% minoxidil for the treatment of androgenetic alopecia.

BRIEF SUMMARY

Described herein are embodiments including point-of-care devices for measuring the SULT 1A1 biomarker contained in one or more plucked human hair(s). The chemistries of the device are arranged in two parts in such a way that they will not spontaneously react. As such, the described device has increased stability and can be stored at room temperature. The described device can be used for measuring sulfotransferase enzyme activity in one or more plucked human hair(s) for the purpose of predicting the effectiveness of the drug minoxidil for the treatment of androgenetic alopecia.

In one embodiment of the current invention, a two-part chemistry composition is prepared such that a reactive agent is isolated and stabilized. In one embodiment, the reactive agent is potassium *p*-nitrophenyl sulfate (PNPS). In one embodiment, one part of a two-part chemical composition (PART A) is prepared by adding PNPS to a stabilizing solution. In one embodiment, a water solution containing a pH 9 to pH 11 buffering agent is used to stabilize PNPS. In one embodiment, the buffering agent is a carbonate buffer containing sodium bicarbonate and sodium carbonate (pH 10). In another embodiment of the present invention, an aprotic polar solvent is used to stabilize a PNPS solution. In yet another embodiment, the aprotic polar solvent is acetonitrile. In another embodiment, a protic non-aqueous solvent is used to stabilize PNPS. In another embodiment, a protic non-aqueous solvent is isopropanol or ethanol.

In one embodiment of the current invention, one part of a two-part chemical composition (PART A) is deposited in a reaction vessel. In another embodiment, the solvent is removed leaving only the stabilized solutes of PART A. In another embodiment, the solvent is removed by air drying and vacuum. In yet another embodiment, a benchtop concentrator (e.g., speedvac) is used to remove solvent from a stabilized PART A solution. In yet another embodiment, lyophilization is used to remove the solvent.

In one embodiment of the present invention, a 50 mM PNPS solution is stabilized in a 10 mM carbonate buffer (pH 10). In yet another embodiment, 10 $\mu$L of a 50 mM PNPS solution stabilized in a 10 mM carbonate buffer (pH 10) is added to a reaction vessel (e.g., a 500 $\mu$L tube) and the water is evaporated under vacuum. In another embodiment, a benchtop concentrator (speedvac) is used to remove water from a stabilized PNPS solution.

In one embodiment of the present invention, the second part of a two-part chemical composition (PART B) is prepared by adding all non-reactive ingredients to a solution. In one embodiment, the second part of a two-part chemical composition contains 50 mM phosphate buffer (pH 8), 100 $\mu$M minoxidil and 5 mM $MgCl_2$.

In one embodiment of the present invention, a kit is prepared containing a PART A and PART B of a two-part chemical composition. In another embodiment, the PART A and PART B components are combined to produce a functional device. In yet another embodiment, a functional device created by combining the PART A and PART B components is used to test for the presence of an enzyme. In yet another embodiment, the enzyme is SULT1A1.

In one embodiment, devices as described above may be used for the predictive assessment of an individual's likelihood to respond favorably to the drug minoxidil. Minoxidil requires biochemical activation by minoxidil sulfotransferase to form the active minoxidil sulfate metabolite. The exact mechanism of action for minoxidil based treatment of androgenetic alopecia is not completely understood. However, *in vitro* studies have demonstrated that minoxidil sulfate is the active metabolite of minoxidil. Response to minoxidil for the treatment of androgenetic alopecia has been associated with differences in scalp sulfotransferase activity, particularly sulfotransferase 1A1 (SULT 1A1). Therefore, a subject with a high level of SULT 1A1 activity will generate more minoxidil sulfate, and therefore will likely have a good response to minoxidil for the treatment of androgenetic alopecia. On the other hand, a subject with a low level of SULT 1A1 activity will not generate much minoxidil sulfate and will likely have a poor response to minoxidil for the treatment of androgenetic alopecia.

In accordance with one approach described herein, a subject's hair follicle sample may be obtained. Preferably, at least two hair follicles may be obtained, so that if only one is analyzed, there will be at least one backup if needed. In one embodiment of the present invention one hair is added to a functional device created by combining the PART A and PART B components of a two-part chemistry. In yet another embodiment two hair follicles are added. In yet another embodiment, at least 3 hair follicles are added to a functional device.

In one embodiment, this reaction may take place in a container other than those specifically described herein. For example, it may be a transparent container with a lid or other opening in which the hair follicle samples may be inserted. In one non-limiting example, the total amount of liquid in the assay container may be about 0.1 ml.

As part of the above reaction, it is understood that in the presence of minoxidil sulfotransferase activity, *p*-nitrophenyl sulfate is converted to the colorimetric *p*-nitrophenol.

The reaction may, in one embodiment, be mixed and then incubated for approximately 4 to 24 hours at room temperature depending on the number of hair follicles used in the assay. Mixing may be by any mixing means known in the art, including shaking the container. In one embodiment, a shorter incubation time may be required for a greater number of hair follicles. In one embodiment, an assay that uses one hair follicle may be incubated for approximately 24 hours. In another embodiment, an assay that uses two hair follicles and may be incubated for approximately 16 hours.

After incubation, the absorbance of *p*-nitrophenol may be read at about 405 nm with a spectrophotometer. The absorbance may be interpreted as follows: (a) absorbance less than 0.4 absorbance units (AU) means minoxidil will not likely work; (b) absorbance equal to 0.4 AU means equivocal test; or (c) absorbance greater than 0.4 AU means minoxidil may work. Alternatively, the color intensity of *p*-nitrophenol may be compared to a reference color card with a range of intensities corresponding to SULT 1A1 activity. Subjects with a relatively high level of sulfotransferase activity will have a relatively strong colorimetric readout, resulting in a relatively significant color change. In comparison, patients with a relatively low level of sulfotransferase activity will have a relatively weak colorimetric readout, and correspondingly, a relatively minimal color change. Patients with a strong colorimetric assay response would be expected to respond to minoxidil for hair re-growth or retention. Whereas, patients with a weak colorimetric assay response would be expected to have a poor response to minoxidil.

## BRIEF DESCRIPTION OF THE DRAWINGS

The above and other objects, aspects, features, advantages and possible applications of the present innovation will be more apparent from the following more particular description thereof, presented in conjunction with the following drawings.

FIG. 1 shows an exemplary kit that may be used for performing colorimetric assays with plucked human hair.
FIG. 2 is an exemplary representation of the PART A and PART B components of an exemplary two-part chemistry composition.
FIG. 3 is an exemplary reaction scheme of the assay.

## DETAILED DESCRIPTION

Examples are described herein in the context of a point-of-care diagnostic device for measuring biomarkers from plucked human hair. The following description is illustrative only and is not intended to be in any way limiting. Other embodiments will readily suggest themselves to such skilled persons having the benefit of this disclosure. Reference will now be made in detail to implementations of the example embodiments.

In the interest of clarity, not all of the routine features of the implementations described herein are shown and described. In the development of any such actual implementation, numerous implementation-specific decisions must be made in order to achieve the developer's specific goals, such as compliance with application- and business-related constraints, and that these specific goals will vary from one implementation to another and from one developer to another. Moreover, such a development effort might be complex and time-consuming but would nevertheless be a routine undertaking of engineering for those of ordinary skill in the art having the benefit of this disclosure.

The term "exemplary" is used exclusively herein to mean "serving as an example, instance or illustration." Any embodiment described herein as "exemplary" is not necessarily to be construed as preferred or advantageous over other embodiments.

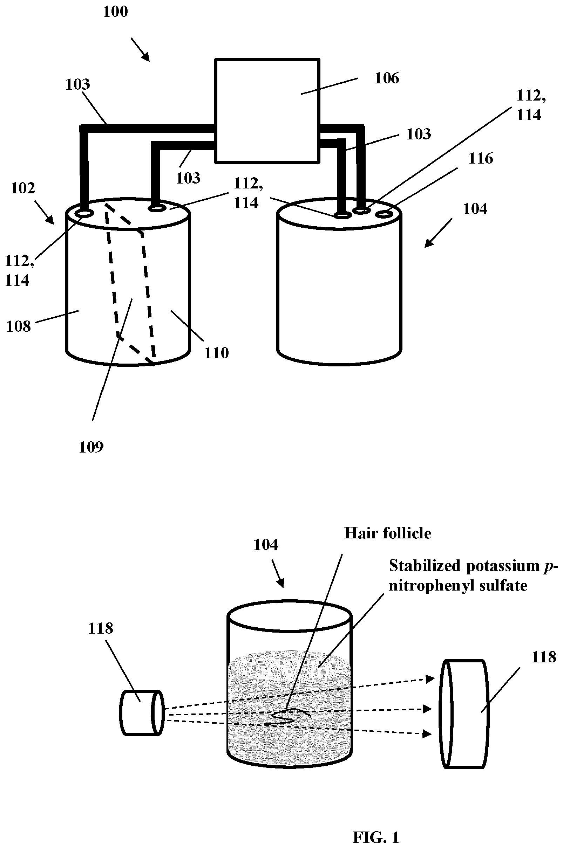

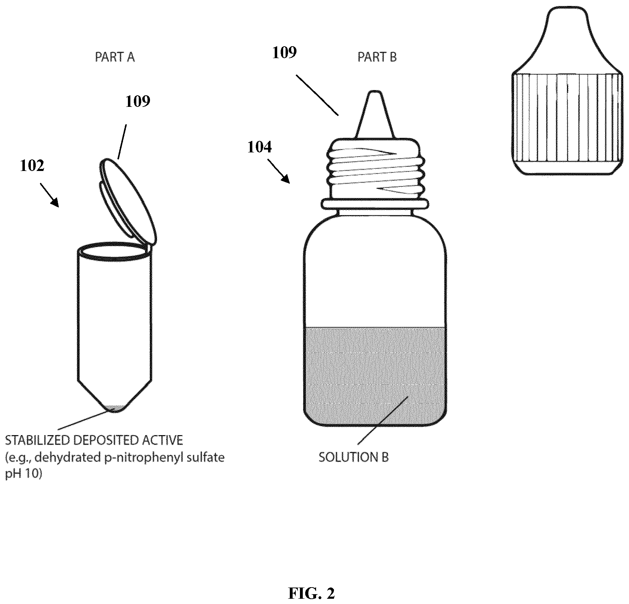

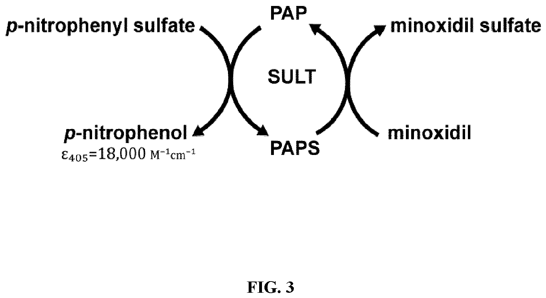

Referring to FIGS. 1-3, embodiments relate to a kit 100 for measuring sulfotransferase enzyme activity in a hair follicle. The kit 100 can include a container 102 and a reaction vessel 104. The kit 100 can include the container 102 and the reaction vessel 104 as separate components. For instance, the kit 100 can include the container 102 as one unit and the reaction vessel 104 as a separate and distinct unit. In the alternative, the kit 100 can include the container 102 and the reaction vessel 104 as part of a single component. For instance, the kit 100 can include a housing or other structure that includes the container 102 and the reaction vessel 104 as a single unit. The kit 100 can include any number of containers 102 and/or reaction vessels 104. There can be one container 102 for one or more reaction vessels 104, an individual container 102 for each individual reaction vessel 104, etc. As will be explained herein, contents of the container 102 will be introduced into the reaction vessel 104. This can be done by pouring the contents of the container 102 into the reaction vessel 104, allowing (e.g., gravity fed, osmosis movement, capillary action movement, etc.) the contents to flow via conduit 103 from the container 102 to the reaction vessel 104, forcing (e.g., via a pump, non-equilibrium kinetics movement, etc.) the contents to flow via conduit 103 from the container 102 to the reaction vessel 104, etc. The conduit 103 can be flow tubes or flow paths (which may be formed in the housing or other structure if the container 103 and reaction vessel 104 are a single unit) that includes the container 102 and the reaction vessel 104, for example. The conduit 103 can be configured to place the container 102 in fluid communication with the reaction vessel 104. Some embodiments can include an actuator 106 (e.g., a valve, solenoid valve, etc.) to control the flow of contents from the container 102 to the reaction vessel 104. The actuator 106 can include a processor, control module, switch, etc. configured to control aspects of the pump, valve, etc. The actuator 106 can be mechanically operated, electro-mechanically operated, etc. Other aspects of the kit 100 can include membranes, filters, gaskets, switches, timers, etc. to facilitate proper control and flow of contents, control of flow rate and amounts, control of flow start and stop times, etc. Thus, the kit 100 can be configured such that the container 102 is in selective fluid communication, via the actuator 106, with the reaction vessel 104 such that the contents of the reactive portion 108 and the contents of the non-reactive portion 110 are introduced into the reaction vessel 104 upon actuation of the actuator 106. It is understood that there may be one or more actuators 106 for a given kit 100.

The container 102 can be configured to hold a two-part chemistry composition. The two-part chemistry composition can have a reactive portion 108 and non-reactive portion 110. For instance, the container 102 can be a container (e.g., bottle, vessel, tank, flask, etc.) with a bifurcated structure 109 forming a reactive cavity and a non-reactive cavity, each cavity configured to hold and retain contents (e.g., chemical composition). It is contemplated for the contents of each portion 108, 110 to be fluids. The bifurcated structure 109 can be a wall configured to physically and/or chemically separate the contents of the reactive portion 108 and the contents of the non-reactive portion 110. The material of the container 102 can physically and/or chemically isolate the contents of the reactive portion 108 from the non-reactive portion 110 and from the environment outside the container 102 - e.g., prevent permeable movement, prevent light from entering, prevent contents of each from mixing, etc. The material of the container 102 can physically and/or chemically isolate the contents of the non-reactive portion 110 from the reactive portion 108 and from the environment outside the container 102 - e.g., prevent permeable movement, prevent light from entering, prevent contents of each from mixing, etc.

The container 102 and the reaction vessel 104 can each include an inlet 112 and an outlet 114 to facilitate ingress and egress of contents. The container 102 may have an individual inlet 112 for each portion 108, 110, a separate individual outlet 114 for each portion 108, 110, etc. In some embodiments, the inlet 112 serves as both the inlet 112 and the outlet 114. The reaction vessel 104 may have an inlet 112 and an outlet 114, or its inlet 112 may serve as both the inlet and outlet 114. Any of the inlets 112 or outlets 114 of the container 102 or the reaction vessel 104 can have a lid 109 or plug to selectively cover the inlet 112 or outlet 114. With embodiments in which the container 102 is separate from the reaction vessel 104, a user can handle the container 102 in their hand and pour to contents from each portion 108, 110, via the outlet 114, into the reaction vessel 104 inlet 112. In addition, or in the alternative, a transfer device, such as a pipette droplet, can be used to transfer the contents - e.g., a user can introduce the pipette into the outlet 114 of the container 102 to retrieve a volume of the contents and dispense the contents into the reaction vessel 104 inlet 112. Also, a pump with conduit 103 lines (extending to and from the inlets 112 and outlets 114) can be used to transfer the contents from the container 102 to the reaction vessel 104. With embodiments in which the container 102 is part of the same unit as the reaction vessel 104, actuator 106 can be actuated to cause/allow flow of contents from the container 102 outlet 114 to the reaction vessel 104 inlet 112.

It is contemplated for the contents of the reactive portion 108 to not mix or combine with the contents of the non-reactive portion 110 until the contents of each are introduced into the reaction vessel 104. Thus, the methods of content transfer, the arrangement and configuration of conduit 103, transfer devices, valves, actuators 106, etc. can be such that no mixing or combining of the contents occurs until they are introduced into the reaction vessel 104.

The reaction vessel 104 can be configured for combining or mixing contents of the reactive portion 108 and contents of the non-reactive portion 110. For instance, the reaction vessel 104 can be a vessel having a cavity that receives the contents of the container 102 when the contents are allowed/forced to flow from the container 102 to the reaction vessel 104. The cavity allows for combining or mixing of the reactive portion 108 contents and the non-reactive portion 110 contents. This can be via free mixing as the contents enter the reaction vessel 104, forced mixing, other forms of agitation, etc.

It is contemplated for the contents of the reactive portion 108 to include potassium *p*-nitrophenyl sulfate. The contents of the reactive portion 108 can also include a stabilizing solution. The stabilizing solution stabilizes potassium *p*-nitrophenyl sulfate to form stabilized potassium *p*-nitrophenyl sulfate. A hydrogen is required to convert potassium p-nitrophenyl sulfate (PNPS) to potassium *p*-nitrophenyl (PNP) and $SO_3$. Having a basic pH minimizes the amount of available protons and stabilized PNPS. The stabilizing solution can have a pH within a range from about 9 to about 11. In some embodiments, the stabilizing solution can be a solvent comprising a water solution and a buffering agent. The buffering agent can be a carbonate buffer comprising sodium bicarbonate and sodium carbonate. In some embodiments, the stabilizing solution can be a solvent comprising aprotic polar solvent. The aprotic polar solvent can comprise acetonitrile. In some embodiments, the stabilizing solution can be a protic non-aqueous solvent. The protic non-aqueous solvent can comprise isopropanol or ethanol.

It is contemplated for the contents of the non-reactive portion 110 to include minoxidil, magnesium chloride, and phosphate buffer. The SULT1A1 enzymatic reaction is optimized at about pH 8, and thus the phosphate buffer can have a pH of about 8.

The reaction vessel 104 can include an opening 116 configured to receive at least one hair follicle. This opening 116 can be the same as the reaction vessel 104 inlet in some instances. The reaction vessel 104 can be configured to receive at least a portion of the hair follicle (e.g., only a portion of the hair follicle is inserted within the reaction vessel 104) or the entire hair follicle (e.g., the entire hair follicle is inserted within the reaction vessel 104). It is contemplated for the hair follicle to be placed into contact with the mixture or combination of contents (the mixture or combination of reactive portion 108 contents and non-reactive portion contents). This can include making contact with the mixture or combination, being submerged within a bath of the mixture or combination, etc. The reaction vessel 104 can be configured to receive more than one hair follicle, which can be via one opening 116 or via multiple openings 116.

Embodiments can relate to a method for determining whether a minoxidil formulation will be effective in a subject in need of hair-regrowth or maintenance. The method can involve combining or mixing contents of a reactive portion 108 and contents of a non-reactive portion 110 of a two-part chemistry composition to form a mixture or combination. Contents of the reactive portion can include potassium *p*-nitrophenyl sulfate. The contents of the reactive portion 108 can also include a stabilizing solution. The stabilizing solution stabilizes potassium *p*-nitrophenyl sulfate to form stabilized potassium *p*-nitrophenyl sulfate. The stabilizing solution can be a solvent comprising a water solution and a buffering agent, an aprotic polar solvent, or a protic non-aqueous solvent. It is contemplated using pure solvent for the aprotic polar or protic non-aqueous solvents. For the water based solution, it is contemplated using 50 mM carbonate buffer. The range can be from 10 to 100 mM and from 100 mM to 1M buffer; however, the lowest amount possible to stabilize PNPS is preferred. The contents can include stabilized potassium *p*-nitrophenyl sulfate in which the solvent is removed so as to leave only stabilized solutes. The solvent can be removed (e.g., 90-100% is removed) via air drying, vacuum, e.g., using a benchtop concentrator, lyophilizaton, etc. For instance, the contents can be subjected to 20 minutes of air drying under vacuum (e.g., 20 to 0.1 Torr).

Contents of the non-reactive portion can include minoxidil, magnesium chloride, and phosphate buffer.

The method can involve placing a hair follicle from the subject in contact with the mixture or combination. The method can involve mixing and/or incubating the hair follicle with the mixture or combination. Mixing can involve stirring, use of a magnetic mixer, shaking, other forms of agitation, etc. Mixing and/or incubating can involve mixing and/or incubating for a period of time that depends on a number of hair follicles placed into contact with the mixture or combination. For instance, it is contemplated that the more hair follicles used for given implementation, the less mixing and/or incubation is needed. For instance, the incubation time can be 24 hrs. for 2 hair follicles, 12 hrs. for 4 hair follicles, etc. The volume of contents used in the reaction vessel 104 can also depend on the number of hair follicles placed into contact with the mixture or combination. For instance, the incubation time using a reaction volume of 100 uL would be twice the incubation time if reaction volume of 50 uL is used.

Presence of the *p*-nitrophenol product is positively correlated with sulfotransferase enzyme activity of the hair follicle, and thus the method can involve measuring presence of a *p*-nitrophenol product within the mixture or combination. The method can involve comparing the measured presence of the *p*-nitrophenol product to a predetermined level. The predetermined level can be a threshold level used as an indicator that the subject will have a good response to minoxidil treatment. For instance, a presence of the *p*-nitrophenol product greater than the predetermined level can indicate a level of sulfotransferase enzyme activity signifying that the subject will have a good response to minoxidil treatment. A presence of the *p*-nitrophenol product less than the predetermined level can indicate a level of sulfotransferase enzyme activity signifying that the subject will not have a good response to minoxidil treatment.

In the presence of minoxidil sulfotransferase activity, *p*-nitrophenyl sulfate is converted to the colorimetric *p*-nitrophenol. Thus, measuring presence of a *p*-nitrophenol product can involve measuring absorbance of the *p*-nitrophenol product at about 400-410 nm (preferably 405 nm) using a spectrophotometer 118 (e.g., spectroscopy instrument having a sensor to collect electromagnetic emission of light passing through a solution and a processor for interpreting or quantifying the collected electromagnetic emissions). In an exemplary embodiment, absorbance may be interpreted as follows: (a) absorbance less than 0.4 AU can indicate minoxidil will not likely work; (b) absorbance equal to 0.4 AU can indicate an equivocal test; or (c) absorbance greater than 0.4 AU can indicate minoxidil may work.

In addition, or in the alternative, measuring presence of a *p*-nitrophenol product can involve comparing a color intensity of the *p*-nitrophenol product to a color intensity reference card with a range of intensities corresponding to sulfotransferase enzyme activity. For instance, the color intensity of *p*-nitrophenol may be compared to a reference color card with a range of intensities corresponding to SULT1A1 activity. Subjects with a relatively high level of sulfotransferase activity will have a relatively strong colorimetric readout, resulting in a relatively significant color change (change in color of the mixture due to any of the p-nitrophenyl sulfate converting to colorimetric *p*-nitrophenol). In comparison, subjects with a relatively low level of sulfotransferase activity will have a relatively weak colorimetric readout (change in color of the mixture due to any of the *p*-nitrophenyl sulfate converting to colorimetric *p*-nitrophenol), and correspondingly, a relatively minimal color change. Subjects with a strong colorimetric assay response would be expected to respond to minoxidil for hair re-growth or retention. Whereas, subjects with a weak colorimetric assay response would be expected to have a poor response to minoxidil. The color intensity reference card can be a physical card or virtual card (e.g., computer graphical user interface) that is pre-made based data from previous tests. The comparison of the colorimetric readout to the color intensity reference card can be by visual human inspection, by computer algorithmic inspection, etc.

Hence, if the hair follicle contains SULT1A1 (or a sufficient amount of SULT1A1), *p*-nitrophenyl sulfate is converted to the colorimetric *p*-nitrophenol (or a sufficient amount of *p*-nitrophenyl sulfate is converted to the colorimetric *p*-nitrophenol) so as to indicate a sufficient level of sulfotransferase enzyme activity, which can be used as a predictor that the subject will have a good response to minoxidil treatment.

While embodiments and applications have been shown and described, it would be apparent to those skilled in the art having the benefit of this disclosure that many more modifications than mentioned above are possible without departing from the inventive concepts disclosed herein. The invention, therefore, is not to be restricted except in the spirit of the appended claims.

EXAMPLE 1: Minoxidil Response Test (MRT), Two Part Chemistry Formulation

The example procedure uses the following reagents: sodium phosphate buffer (500 mM), sodium carbonate, sodium bicarbonate, potassium *p*-nitrophenyl sulfate, minoxidil, $MgCl_2$ solution (1 M), water (0.1 $\mu$m filtered) and methanol.

The example procedure is for production of 1000 units of MRT reactions. PNPS deposited sample tubes are prepared (PART A), as well as the reconstituting solution (SOLUTION B). A single MRT reaction unit is created by adding 100 $\mu$L of SOLUTION B to a PNPS deposited sample tube (PART A).

MANUFACTURING PROCEDURE

1.0 Solution 1: PNPS Buffer 10 mM (pH 10)

Table 1 - PNPS Buffer 10 mM (pH 10) Preparation

| Component | Amount per 1 L | Calculated Amount | Actual Amount |
|---|---|---|---|
| Sodium Bicarbonate | 0.388 g | g | g |
| Sodium Carbonate | 0.571 g | g | g |
| Pure Water | 1.0 L | L | L |

Compound the solution according to the calculated amounts indicated in the table as follows: add the pure water to an appropriately sized container, add sodium bicarbonate and sodium carbonate and mix on a magnetic stir plate until dissolved.

Measure the pH of the final solution (pH 10).

2.0 Solution 2: Potassium *p*-Nitrophenyl Sulfate (PNPS) 50 mM (pH 10)

Table 2 - Potassium *p*-nitrophenyl sulfate (PNPS) 50 mM (pH 10) Preparation

| Component | Amount per 5 mL | Calculated Amount | Actual Amount |
|---|---|---|---|
| Potassium *p*-Nitrophenyl Sulfate | 0.128 g | g | g |
| PNPS Buffer | 10.0 mL | mL | mL |

Compound the solution according to the calculated amounts indicated in the table as follows: add PNPS buffer to an appropriately sized container, add potassium p-nitrophenyl sulfate and mix on a magnetic stir plate until dissolved.

3.0 Solution 3 - 100 mM Minoxidil Stock Solution

Table 3 - 100 mM Minoxidil Stock Solution Preparation

| Component | Amount per 5 mL | Calculated Amount | Actual Amount |
|---|---|---|---|
| Minoxidil | 0.105 g | g | g |
| Methanol | 5.0 mL | mL | mL |

Compound the solution according to the calculated amounts indicated in the table as follows: add the methanol to an appropriately sized container, add the minoxidil and mix on a magnetic stir plate until dissolved.
Store stock solution at about -20 °C for up to 1 year.

4.0 PNP Deposited Tube (PART A)

Table 4 - Solution 2 Calculation

| Materials | Amount per | Calculated Amount |
|---|---|---|
| Solution 2 | 10 uL | mL |

The materials in step 4.0 include: Solution 2 (PNPS 50 mM, pH 10) and a sample tube (500 μL).
Perform the following: (1) add 10 μL of Solution 2 to MRT sample tube, (2) place open tube in speedvac and air dry under vacuum with spinning until all liquid has evaporated (approximately 20 min) and (3) store at ambient temperature.
All volumes shall be within 5% of the specification. The minimum time required to evaporate liquid should be determined for each instrument (speedvac) used. Overdrying may reduce stability of PART A.

5.0 Solution B

The materials in step 5.0 include: 500 mM phosphate buffer, pH 8.0; 1 M magnesium chloride solution; 100 mM minoxidil stock solution and water.

Table 5 - Solution B Preparation

| Component | Amount per 100 mL | Calculated Amount | Actual Amount |
|---|---|---|---|
| 500 mM Phosphate Buffer, pH 8.0 | 10 mL | mL | mL |
| 1 M Magnesium Chloride Solution | 0.100 mL | mL | mL |

(continued)

| Component | Amount per 100 mL | Calculated Amount | Actual Amount |
|---|---|---|---|
| 100 mM Minoxidil Solution | 0.500 mL | mL | mL |
| Pure Water | 89.400 mL | mL | mL |

Compound the solution according to the calculated amounts indicated in the table, then mix on a magnetic stir plate until uniform.

All volumes shall be within 5% of specification. All weights shall be within 5% of specification. The pH should be 8 ± 0.5.

EXAMPLE 2: Accelerated Stability Protocol

TEST COMPONENTS

Two (2) verification batches (lots) of potassium $p$-nitrophenyl sulfate (PNPS) deposited tubes were created and stored at elevated temperature. Each lot contains a total of twenty-four (24) PNPS deposited tubes. During the course of the accelerated stability study, 3 samples of each lot will be removed from the elevated temperature and reconstituted with the second part of the two-part chemistry (Solution B). After 24h of reconstitution, samples are visually inspected for yellow color.

JUSTIFICATION FOR MODEL

Subjecting test devices to extreme temperature conditions is intended to simulate product degradation over time, but at an accelerated rate. Using the Arrhenius equation, shelf-life data can be extrapolated as a predictive measurement for product performance. The Arrhenius equation relates chemical reaction rate (k) to absolute temperature ($T$):

$$d(\mathrm{In}k)/d\mathrm{T} = \Delta\mathrm{E}_a/\mathrm{RT}^2$$

Where $\mathrm{E}_a$ is the activation energy and R is the universal gas constant.

Table 6 - Arrhenius Model for MINOXIDIL SENSITIVITY ASSAY

| Treatment Temperature (°C) | Days Required for **1 year** stability @ 23°C | Days Required for **1.5 year** stability @ 23°C | Days Required for **2 year** stability @ 23°C |
|---|---|---|---|
| 50 | 56.2 days | 84.3 days | 112.3 days |
| 40 | 112.3 days | 168.6 days | 224.6 days |

Using the Arrhenius model at 20 kcal, the test reagent would have satisfactory performance theoretically for 1 years if specifications were met after 56.2 days at 50°C, or 112.3 days at 40°C.

NOTE: The 40°C samples are run as a contingency in the event that the 50°C conditions are too extreme for the test samples. In the event that test samples remain stable for 56.2 days at 50°C, the 40°C experiment is terminated.

PRESUMED STORAGE CONDITIONS

The performance of the PNPS deposited tubes is evaluated when stored at 50°C and 40°C. All storage locations conform to Applied Biology QSR to assure appropriate temperature ranges are met during the study.

OBJECTIVE AND PURPOSE OF TESTING

The purpose of stability testing at 50°C and 40°C is to generate data to determine the shelf life of the product. The data is used as a predictor of stability for the final product form.

TEST KIT STORAGE CONDITIONS

Kits (reagent) is stored at 50°C and 40°C ($\pm$ 2°C for all temperatures).

INTERVAL BETWEEN ANALYSES

Table 7 - MINOXIDIL SENSITIVITY ASSAY Accelerated Stability Study

| Storage Temp | Duration |
|---|---|
| 50°C | Day 6, 14, 28, 42, 56, 70, 84, 98 |
| 40°C | Day 6, 14, 28, 70, 98, 126, 154, 182 |

An accelerated stability is carried out on the MINOXIDIL SENSITIVITY ASSAY reagent stored at 50°C $\pm$ 2°C and 40°C $\pm$ 2°C as described in Table 7. Reagent is tested in triplicate. If a testing day falls on a weekend or holiday, the test point is either eliminated (depending on the testing interval) or tested on the first workday following the scheduled time.

STABILITY CRITERIA

The accelerated stability studies must initially pass the acceptance targets detailed in Table 8.

TABLE 8 - MINOXIDIL SENSITIVITY ASSAY Specificity Release Testing and Stability Specifications

| Description | Result Specification (visual) | Result Determination |
|---|---|---|
| PNPS deposited tubes + 100 uL Solution B | Clear | Pass |
| PNPS deposited tubes + 100 uL Solution B | Slight Yellow | Pass |
| PNPS deposited tubes + 100 uL Solution B | Yellow | Fail |

NOTES:

(1) Results are evaluated 24 hours after reconstitution with Solution B.
(2) Reagent must meet the specifications below (#4) for 56.2 days at 50°C or 112.3 days at 40°C to yield a projected 12-month shelf life at 23°C.
(3) A failure at a specific temperature may not be an indicator of the shelf life since higher temperatures may adversely affect the solution chemistry. Thus, a 1 year shelf life may not be projected at the 50°C treatment, where it may be projected at the 40°C treatment.
(4) A time point is deemed a failure when fail is observed on two (2) consecutive testing time points. The failure time may defer from that of the first time point failure. If there is an invalid test result, the test may be retested once to confirm the result. A failure may also be retested once to confirm the result. If upon retest, the failure is not confirmed, an additional test may be run to assess the status of the time point.

ATTACHMENT A

| | 0 | 6 | 14 | 28 | 42 | 56 | 70 | 84 | 98 | 126 | 154 | 182 | Total Vials |
|---|---|---|---|---|---|---|---|---|---|---|---|---|---|
| 50 | | ✓ | ✓ | ✓ | ✓ | ✓ | ✓ | ✓ | ✓ | | | | 24 |
| 40 | | ✓ | ✓ | ✓ | | | ✓ | | ✓ | ✓ | ✓ | ✓ | 24 |

NOTES:

Samples shall be evaluated in triplicate.

If a testing day falls on a weekend or holiday, the test point is eliminated (depending on the testing interval) or tested on the first work day following the scheduled time.

1 Year Estimated stability: 50°C = 56.2 days, 40°C = 112.3 days

RESULTS

Table X - Accelerated Stability Report for Formula (pH 7.5-9.5)

| Days | 0 | 6 | 14 | 28 | 42 | 56 | 70 | 84 | 98 | 126 | 154 | 182 |
|------|-----|------|------|------|------|------|------|------|------|------|------|------|
| **50C** | Pass | Pass | Fail | NA | NA | NA | NA | NA | NA | NA | NA | NA |
| **40C** | Pass | Pass | Pass | Pass | Pass | Pass | Pass | Fail | Fail | NA | NA | NA |

**Formula G2 (pH 7.5-9.5)**

Table Y - Accelerated Stability Report for Formula (pH 9.5-10.5)

| Days | 0 | 6 | 14 | 28 | 42 | 56 | 70 | 84 | 98 | 126 | 154 | 182 |
|------|------|------|------|------|------|------|------|------|------|------|------|------|
| **50C** | Pass | Pass | Pass | Pass | Pass | Pass | Pass | Pass | Pass | Pass | Pass | Pass |
| **40C** | Pass | Pass | Pass | Pass | Pass | Pass | Pass | Pass | Pass | Pass | Pass | Pass |

EXAMPLE 3: Functional Testing of Reconstituted PART A and PART B

Samples deemed stable from Example 2, i.e., those that remained clear after 24h of reconstitution, were reacted with two human hair follicles to test whether the reconstituted chemistries were functional.

Specifications

| Description | Result Specification (visual) | Result Determination |
|-------------|-------------------------------|----------------------|
| PNPS deposited tubes + 100 uL Solution B + Hair | Clear | Fail |
| PNPS deposited tubes + 100 uL Solution B + Hair | Yellow | Pass |

RESULTS

Table X - Accelerated Stability Report for Formula (pH 7.5-9.5)

| Days | 0 | 6 | 14 | 28 | 42 | 56 | 70 | 84 | 98 | 126 | 154 | 182 |
|------|-----|------|------|------|------|------|------|------|------|------|------|------|
| **50C** | Pass | Pass | NA | NA | NA | NA | NA | NA | NA | NA | NA | NA |
| **40C** | Pass | Pass | Pass | Pass | Pass | Pass | Pass | NA | NA | NA | NA | NA |

**Formula G2 (pH 7.5-9.5)**

Table Y - Accelerated Stability Report for Formula (pH 9.5-10.5)

| Days | 0 | 6 | 14 | 28 | 42 | 56 | 70 | 84 | 98 | 126 | 154 | 182 |
|------|---|---|----|----|----|----|----|----|----|-----|-----|-----|
| 50C | Pass | Pass | Pass | Pass | Pass | Pass | Pass | Pass | Pass | Pass | Pass | Pass |
| 40C | Pass | Pass | Pass | Pass | Pass | Pass | Pass | Pass | Pass | Pass | Pass | Pass |

**Claims**

1. A kit for measuring sulfotransferase enzyme activity in a hair follicle comprising:

   a container configured to hold a two-part chemistry composition, the two-part chemistry composition having a reactive portion and non-reactive portion; and
   a reaction vessel configured for combining or mixing contents of the reactive portion and contents of the non-reactive portion;
   wherein contents of the reactive portion include potassium *p*-nitrophenyl sulfate;
   wherein contents of the non-reactive portion include minoxidil, magnesium chloride, and phosphate buffer.

2. The kit of claim 1, wherein the container is configured to hold the contents of the reactive portion in physical and/or chemical isolation from the contents of the non-reactive portion.

3. The kit of claim 1, wherein contents of the reactive portion include a stabilizing solution.

4. The kit of claim 1, wherein the stabilizing solution stabilizes potassium p-nitrophenyl sulfate to form stabilized potassium p-nitrophenyl sulfate.

5. The kit of claim 4, wherein the stabilizing solution is a solvent comprising:

   a water solution and a buffering agent;
   an aprotic polar solvent; and/or
   a protic non-aqueous solvent.

6. The kit of claim 5, wherein the stabilizing solution has a pH within a range from about 9 to about 11; or

   wherein the buffering agent is a carbonate buffer comprising sodium bicarbonate and sodium carbonate; or
   wherein the aprotic polar solvent comprises acetonitrile; or
   wherein the protic non-aqueous solvent comprises isopropanol or ethanol.

7. The kit of claim 1, wherein the phosphate buffer has a pH of about 8.

8. The kit of claim 1, wherein the container is in selective fluid communication, via an actuator, with the reaction vessel such that the contents of the reactive portion and the contents of the non-reactive portion are introduced into the reaction vessel upon actuation of the actuator.

9. The kit of claim 1, wherein the reaction vessel includes an opening configured to receive a hair follicle.

10. A method for determining whether a minoxidil formulation will be effective in a subject in need of hair-regrowth or maintenance, the method comprising:
    combining or mixing contents of a reactive portion and contents of a non-reactive portion of a two-part chemistry composition to form a mixture or combination, wherein:

    contents of the reactive portion include potassium p-nitrophenyl sulfate; and
    contents of the non-reactive portion include minoxidil, magnesium chloride, and
    phosphate buffer; and
    placing a hair follicle from the subject in contact with the mixture or combination.

**11.** The method of claim 10, further comprising:

measuring presence of a p-nitrophenol product, wherein the presence of the p-nitrophenol product is positively correlated with sulfotransferase enzyme activity of the hair follicle; or

mixing and/or incubating the hair follicle with the mixture or combination.

**12.** The method of claim 11, further comprising:

comparing the measured presence of the p-nitrophenol product to a predetermined level; and

using the comparison as an indicator that the subject will have a good response to minoxidil treatment.

**13.** The method of claim 12, wherein:

a presence of the p-nitrophenol product greater than the predetermined level indicates a level of sulfotransferase enzyme activity signifying that the subject will have a good response to minoxidil treatment.

**14.** The method of claim 11, wherein:

mixing and/or incubating the hair follicle with the mixture or combination involves mixing and/or incubating for a period of time that depends on a number of hair follicles placed into contact with the mixture or combination.

**15.** The method of claim 11, wherein:

measuring presence of a p-nitrophenol product involves measuring absorbance of the p-nitrophenol product at about 405 nm using a spectrophotometer; and/or

measuring presence of a p-nitrophenol product involves comparing a color intensity of the p-nitrophenol product to a color intensity reference card with a range of intensities corresponding to sulfotransferase enzyme activity.

FIG. 1

PART A

109

102

STABILIZED DEPOSITED ACTIVE
(e.g., dehydrated p-nitrophenyl sulfate
pH 10)

109   PART B

104

SOLUTION B

**FIG. 2**

p-nitrophenyl sulfate $\quad$ PAP $\quad$ minoxidil sulfate

SULT

p-nitrophenol $\quad$ PAPS $\quad$ minoxidil
$\varepsilon_{405}=18{,}000 \ \mathrm{M}^{-1}\mathrm{cm}^{-1}$

**FIG. 3**

Europäisches
Patentamt

European
Patent Office

Office européen
des brevets

# EUROPEAN SEARCH REPORT

Application Number

EP 22 18 0319

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 213 139 530 U (NANFANG HOSPITAL SOUTHERN MEDICAL UNIV) 7 May 2021 (2021-05-07) * figure 1: 331-333, 431-433; translation: description, claims * | 1-15 | INV. C12Q1/48 |
| A | US 2014/335537 A1 (GOREN ANDY OFER [US] ET AL) 13 November 2014 (2014-11-13) | 1-15 | |
| A | MARHOL PETR ET AL: "Preparation of silybin and isosilybin sulfates by sulfotransferase fromDesulfitobacterium hafniense", JOURNAL OF MOLECULAR CATALYSIS B : ENZYMATIC,, vol. 89, 20 December 2012 (2012-12-20), pages 24-27, XP028984906, ISSN: 1381-1177, DOI: 10.1016/J.MOLCATB.2012.12.005 | 1-15 | |
| A | CHEN HSU-CHING ET AL: "Role of SulP, a nuclear-encoded chloroplast sulfate permease, in sulfate transport and H-2 evolution in Chlamydomonas reinhardtii", PHOTOSYNTHESIS RESEARCH, SPRINGER NETHERLANDS, DORDRECHT, NL, vol. 84, no. 1-3, 1 June 2005 (2005-06-01), pages 289-296, XP002368668, ISSN: 0166-8595, DOI: 10.1007/S11120-004-7157-Y | 1-15 | **TECHNICAL FIELDS SEARCHED (IPC)** C12Q G01N |

The present search report has been drawn up for all claims

| Place of search | Date of completion of the search | Examiner |
|---|---|---|
| Munich | 1 March 2023 | Behrens, Ralf |

EPO FORM 1503 03.82 (P04C01)

Europäisches
Patentamt

European
Patent Office

Office européen
des brevets

**Application Number**

**EP 22 18 0319**

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

| | | |
|---|---|---|
| Europäisches Patentamt European Patent Office Office européen des brevets | **LACK OF UNITY OF INVENTION**<br>**SHEET B** | **Application Number**<br>**EP 22 18 0319** |

The Search Division considers that the present European patent application does not comply with the requirements of unity of invention and relates to several inventions or groups of inventions, namely:

```
1. claims: 1-15(partially)

        Kit of claim 1, comprising
        a container holding a two-part chemistry composition, the
        two-part chemistry composition having a reactive portion and
        non-reactive portion; and
        a reaction vessel configured for combining or mixing
        contents of the reactive portion and contents of the
        non-reactive portion;
        wherein contents of the reactive portion include potassium
        p-nitrophenyl sulfate;
        wherein contents of the non-reactive portion include
        minoxidil, magnesium chloride,and phosphate buffer.
        Method of using the kit.
                    ---


2. claims: 1-15(partially)

        Other kits falling in the scope of claim 1. Their use.
                    ---
```

## ANNEX TO THE EUROPEAN SEARCH REPORT
## ON EUROPEAN PATENT APPLICATION NO.

EP 22 18 0319

This annex lists the patent family members relating to the patent documents cited in the above-mentioned European search report.
The members are as contained in the European Patent Office EDP file on
The European Patent Office is in no way liable for these particulars which are merely given for the purpose of information.

01-03-2023

| Patent document cited in search report | Publication date | Patent family member(s) | Publication date |
|---|---|---|---|
| CN 213139530 U | 07-05-2021 | NONE | |
| US 2014335537 A1 | 13-11-2014 | NONE | |

EPO FORM P0459

For more details about this annex : see Official Journal of the European Patent Office, No. 12/82

**REFERENCES CITED IN THE DESCRIPTION**

**Patent documents cited in the description**

- US 10633688 B

- US 8691518 B